# EUROPEAN PATENT SPECIFICATION

(11) **EP 2 303 162 B1**
(45) Date of publication and mention of the grant of the patent: **14.12.2011**
(21) Application number: 08813777.3
(22) Date of filing: 25.07.2008
(51) Int. Cl.: A61B 17/70

(54) **POSTERIOR DYNAMIC SCREW**
POSTERIORE DYNAMISCHE SCHRAUBE
VIS DYNAMIQUE POSTÉRIEURE

(43) Date of publication of application: 06.04.2011
(73) Proprietor: Hays Saglik Urunleri Ic Ve Dis Ticaret Hayvancilik Limited Sirketi, 06560 Ankara (TR)
(72) Inventor: TABUKTEKIN, Ugur, 06560 Ankara (TR)
(74) Representative: Iskender, Ibrahim
(86) International application number: PCT/TR2008/000087
(87) International publication number: WO 2010/011196

(56) References cited:
- DE-U1- 20 207 851
- US-A1- 2005 192 571
- US-A1- 2006 200 131
- US-A1- 2007 161 996
- US-A1- 2008 015 576

## Description

### TECHNICAL FIELD

The invention relates to the posterior dynamic screw, which is developed to provide stabilisation while preventing the damages likely to occur in the vertebrae in the chronic instability cases.

The invention relates in particular to the posterior dynamic screw, which is developed for the stabilization of the joint by means of the discs and facet Joints in the most suitable position so that the daily life will not be negatively affected, in order to eliminate the ailments emerging the thoracal and lumbar region vertebrae.

### PRIOR ART

The beginning of the modern spinal surgery in use today dates back to the 1960's. The foundations of today's modem spinal surgery were laid by the spinal column instrumentation device developed by Harrington under his own name. The fact that said developed device provided correction on a single plane necessitated the improvements to be performed in this field. In line with this need, the transpedicular screws and the corresponding technique have been developed, with the capability of control on three dimensions and with differing properties and designs. The aim is to eliminate the ailment without destroying the motion, hence the arthrodesis therapy is expected to remain on the agenda only for the treatment of the overt instability in future, while artroplasty is expected to be in the focus in cases of chronic instability. The fundamental problem In the glacial instability is the gradual reduction in the capacity of the spinal column to endure the daily loads, as a result of the anatomic integrity being gradually impaired. For the treatment of this case, it is necessary first of all to replace the lost capacity, rather than to destroy the motion that causes the pain. For this purpose, dynamic posterior transpedicular stabilization method is employed. Afterwards, the source (disc) causing the pain is removed, while preserving the motion and applying the therapy by means of total disc prosthesis or artificial nucleus. Although said screws developed provide a solution for the existing problems in the spinal surgery, they also lead to the emergence of different complications, thereby causing the emergence of new problems in the technique that require solution. Implant failure is the most readily recognizable of these complications. Fractures occur especially in the pedicle screws and rods in time, as a result of the metal fatigue. In addiction to these fracture, it may also be possible for the screw to slip off and get out of bone. Moreover, the factors such as the long operation time and the high width of the incision area during the stabilization procedure carried out with transpedicular screws in use today lead to the increased infection risk. Consequently, the inflection that develops after the operation causes the practice of the antibiotic treatment. As a result, the period during which the patient remains hospitalized after the operation becomes considerably extended. All said process increases the treatment costs to a great extent.

At the present, the implants used to treat the spinal column disorders and to prevent the pains are connected on both sides of the spinal column. The connection between said dynamic systems is provided by means of the rods placed parallel to the spinal column. Connection of each said rod with the spinal column is performed by means of the connecting members. Once these connecting members are plated in the system, they are supported by the bone grafts and the fusion of several vertebrae so that they can be locked and any fracture may be prevented. The disadvantage with this technique is that the bone grafts and the fusion of the vertebrae generally lead to the emergence of the complications during the lifetime of the individual, as a result of the healthy disc being subjected to excessive load due to the production of a semirigid transition between the healthy vertebra and the fixed vertebra. In the implantable spinal column tool kit with no infusion, which is developed to bear greater loads without resorting to the bone grafts and the vertebra fusions and is the subject of the U.S. Patent no. 5 672 175, the rods are connected with the spinal column by means of fixing members with fixed center and three dynamic fixing members. Each of said fixing members located at either end is rigidly connected with the connecting member that is connected with a corresponding proper implantable rod in a sliding manner. Even though said invention appears to enable the elimination of the spinal column disorders and to provide freedom of movement to the individual after the operation, it remains insufficient in this regard. After the connection of the implant with the spinal column, a new bone formation may occur between the implant body and the spinal column, and may eliminate the freedom of movement after a certain time.

As an example of the posterior dynamic systems in use today, the mention may be made of application no. 2006/07373 titled "movable dynamic system for the treatment of the spiral column disorder". The movable, dynamic, implantable spinal column tool according to said application comprises at least one fixed support connected with an improvement rod and at least one movable carrier mounted in a sliding manner to the improvement rod. Each of the fixed support and the movable carrier composes a body and a pedicle screw or a transversal protrusion hook joined to the body in an articulated manner. Distribution of the degrees of freedom between the carrier and the rod and the pedicle screws or the hooks and the carrier and the fixed support provides a non-rigid mechanism, which preserves a part of the natural movements of the vertebrae and the disc and the potential growth of the spinal column.

Another example of the posterior dynamic systems in use today, the mention may be made of application no. US 2006200131 (A1), titled "constrained motion bone screw assembly" is A bone screw assembly includes an anchor portion and a head portion, such as a rod-receiving portion, movably mounted to the anchor portion to allow for controlled angulation between the anchor portion and the head portion. The anchor portion is pivotable in one or more selected directions about an axis relative to the head portion. A restriction member, which may be a rod seat, prevents the anchor portion from pivoting in one or more different direction about another axis relative to the head portion and/or a spiral fixation element received in the head portion. The restriction member may be inserted in the head portion to control direction that the anchor portion pivots relative to the head portion. The restriction member may also serve as a compression member and/or rod seat for seating a spinal rod coupled to the bone screw assembly.

Another example of the posterior dynamic systems in use today, the mention may be made of application no. DE20207851, "Anchoring element for securing a rod on a device for adjusting a human or animal vertebral column on a vertebra" titled relates to an anchoring element for securing a rod of a devise for adjusting a human or animal vertebral column on a vertebra, comprising a retaining means ( 10 ) for receiving the rod, a safety element ( 26 ) placed on the retaining means and working against the rod, a securing element ( 14 ) which can be placed on the body of the vertebra, and a clamping device ( 12 ) which is arranged between the retaining means ( 10 ) and the securing element ( 14 ), comprising a ring-shaped mount (32), a partially Conical-segment shaped bearing ( 28 ) and an intermediate element ( 30 ) which is embedded in the mount ( 32 ) and which engages the bearing, whereby the mounting ( 32 ) is movable in a removed state in relation to the bearing ( 28 ), whereas the mount ( 32 ) is maintained in a clamped state on the bearing ( 28 ) by means of the intermediate element ( 30 ). The mount ( 32 ) is rigidly connected to the retaining means ( 10 ) and the bearing ( 28 ) is rigidly connected to the securing element ( 14 ). In order to enable said type of anchoring element, despite the fact that it is displaceably retained, to transmit relatively large amounts of force from the rod to the body of the vertebra without causing slipping, the bearing ( 28 ) comprises flat guiding surfaces ( 38, 40 ) which are formed laterally on two opposite sides (34, 36), and the intermediate element ( 30 ) is provided with corresponding counter surfaces ( 50, 52).

Although today's dynamic systems developed to treat the spinal column disorders and to provide the freedom of movement for the individual eliminate all the complaints in the beginning, they lead to regression in the treatment after a while, resulting from the structure of the implants. The dynamic screws designed to have movable heads enable at first the elimination of the complaints resulting from the spinal column disorders, but they loose their effect after a while, because of the development of the bone in the cavity that remains between the vertebra and the screw. The bone tissue that forms in the cavity between the spinal column and the bone and that prevents the pressure likely to form due to the contact between the screw and the bone forms a protrusion at the exit point of the system, thereby causing the limitation in the ability of movement in time and the regression of the treatment. In such a case, the relapse of the complaints of the individual caused by the spinal column disorder becomes likely. In order to eliminate such complains, it may be necessary for the individual to undergo a repeated operation. As a result, the formed bone tissue causes the hindrance of the continuity of the treatment.

As et result, the presence of the need for the posterior dynamic screw that eliminates the disadvantages of the screws used according to the state of the art in the posterior transpedicular stabilization methods and the inadequacy of the existing solutions have made it necessary to provide an improvement in the relevant art.

### OBJECT OF THE INVENTION

Based on the mentioned state of the art, the invention alms at solving the aforementioned problems.

The object of the invention is to provide that the bone formation occurring in the surface of the screw in contact with the bone is prevented from negatively affecting the ability of movement during the period following the implantation, owing to the special body design of the posterior dynamic screw extended in the downward direction.

Another object of the invention is to prevent the bone formation between the screw and the spinal column, thereby preserving the ability of movement, owing to the protrusion, which is formed in the lower part of the posterior dynamic screw body extending towards the spinal column and serves as a buffer.

Another object of the invention is to enable an increase in the angle of movement, owing to the channels formed in the lower part of the movable body of the posterior dynamic screw in the direction of movement of the screw.

Another object of the invention is to provide the best stabilization in the cases of chronic instability, owing to the posterior dynamic screw developed.

Another object of the invention is to provide the ability for the screw developed with a special body design to be easily used by the spinal column surgeons, owing to the possibility of using the same surgical techniques and hand tools as the convectional transpedicular systems, by way of approaching from the posterior.

Another object of the invention is to provide compatibility with the physiological movements of the spinal column, owing to the movable body of the posterior dynamic screw having a special design.

Still another object of the invention is to provide the distribution of the load imposed on the impaired disc in the aching lumbar syndrome associated with the degenerative disc disease, owing to the ability of the posterior dynamic screw to move in the axial direction.

Still another object of the invention is to prevent the formation of the aching lumbar syndrome, owing to the posterior dynamic screw that allows movement in the axial direction while preventing the dislocation of the vertebra and excessive movement.

Still another object of the invention is to enable the prevention of the emergence of the complications such as pull-out and fracture depending on the special design and the material of the posterior dynamic screw.

Still another object of the invention is to enable the prevention of the rotation by allowing the movement only in the axial direction after the placement in the vertebra, owing to the specially developed design of the posterior dynamic screw.

Still another object of the invention is to provide the maximum strength against the instantaneous loads imposed on the spinal column, owing to the special design and the material of the posterior dynamic screw.

Yet another object of the invention is to completely eliminate the requirement for the use of the artificial band, thereby enabling to completely eliminate the disadvantages resulting from the loosening likely to take place with time, owing to the rod mechanism of the posterior dynamic screw.

Yet another object of the invention is to provide the possibility to adapt to the rigid instrumentation systems according to the needs, owing to the standard rod mechanism of the posterior dynamic screw.

Yet another object of the invention is to permit minimal level of surgery, owing to the presence of the percutaneous use mechanism.

The structural and characteristic features and all the advantages of the invention will be more clearly understood from the figures provided below and the detailed description written with reference to said figures, hence the evaluation must be made taking into consideration these figures and detailed description.

### BRIEF DESCRIPTION OF THE FIGURES

Figure-1 is the overall schematic view of the posterior dynamic screw in disassembled state.
Figure-2 is the overall schematic front view of the posterior dynamic screw In disassembled state.
Figure-3 is the overall schematic view of the body in disassembled state.
Figure-4 is the overall schematic view of the screw in disassembled state.
Figure-5 is the overall schematic view of the head in disassembled state.
Figure-6 is the overall schematic view of the fixing member in disassembled statue.
Figure-7 is the overall schematic view of the tightening member in disassembled state.
Figure-8 is the overall schematic view of the posterior dynamic screw in assembled state.
Figure-9 is the overall schematic view of the posterior dynamic screw in a state applied on the vertebrae.

### REFERENCE NUMBERS

1. Posterior dynamic screw
2. Body
   2.1. Buffer
   2.2. Head slot
   2.3. Tightening member slot
   2.4. Fixing member slot
3. Screw
   3.1. Head
4. Cap
5. Fixing member
6. Tightening member
7. Vertebra

### DETAILED DESCRIPTION OF THE INVENTION

The present invention relates to the posterior dynamic screw (1), which eliminates the situation in which the vertebra (7) becomes a source of pain, by sharing the load imposed on the impaired vertebra (7), in the case of aching lumbar syndrome associated with the degenerative vertebra (7) diseases that occur as a result of the spinal column disorders, owing to its ability of movement in the axial direction, and comprises the buffer (2.1) section formed in the lower part of its body (2) In order to prevent the bone tissue formed after the operation from negatively affecting the ability of movement.

The posterior dynamic screw (1) shown in Figure-1 is used In the system of the non-fusion technique applied in the stabilization of the disorders in the thoracal and lumbar region vertebrae (7). Said posterior dynamic screw (1) comprises the body (2) with the ability of movement in the axial direction, the screw (3) connected by way of passing through the central part of this body (2), the cap (4) that enables to take the movements of the screw (3) under control, the fixing member (5) connected with the cap (4) to serve the function of providing the stabilization of the posterior dynamic screw (1) on the spinal column and the tightening member (6) that enables to secure said fixing member (5) at the point it is positioned.

The posterior dynamic screw (1) shown in Figure-2 is characterised in that it moves in the axial direction depending on the structure of the cap (4) and the screw (3) connected with the cap (4). Owing to said axial movement, it is allowed to treat the disorder arising in a painful form in the spinal column of the individual without loosing the ability of movement. Owing to the channels formed in the lower part of said body (2) in the direction of movement of the posterior dynamic screw (1), this angle of movement is enabled to be increased.

The body (2), which is illustrated in Figurs-3 and is one of the main components of the posterior dynamic screw (1), is formed with the shape of a tulip. In the lower section of said body (2), a protrusion called buffer (2.1) is formed on the surface facing the vertebra (7). Said buffer (2.1) prevents the bone formation, which occurs on the surface of the posterior dynamic screw (1) that contacts the vertebra (7), from adversely affecting the ability of movement during the period following the implantation. The buffer (2.1) formed on the lower part of the body (2) of said posterior dynamic screw that extends towards the vertebra (7) constrains the bone formation between the posterior dynamic screw (1) and the vertebra (7), thereby enabling the preservation of the ability of movement of the spinal column. The fixing member slot (2.4) is formed on said body (2) such that these will be aligned in the projection of each other. The task of said fixing member slot (2.4) is to enable the fixing member (5) to be seated under best conditions on the screw (3) so that the locking procedure is achieved in a secure manner. The tightening member (6), which is positioned to enable said fixing member (5), cap (4) and the screw (3) to be connected to each other in a secure and reliable manner, is connected by way of engagement into the tightening member slot (2.3) located on the upper part of said body (2). In the middle portion of said body (2), the head slot (2.4) designed with a spherical shape is formed to position the cap (4) and the head (3.1).

Owing to the flat surfaces formed by way of smoothing both sides of the head (3.1) formed in the uppermost section of the screw (3), which screw (3) is shown in Figure-4 and is connected by way of entering the vertebra (7), the cap (4) is enabled to fit onto the screw (3), while at the same time enabling to provide the screw (3) with the ability of movement only in the axial direction. Gaps are left between said cap (4) and the smoothened surfaces located on the head (3.1) section of this screw (3) according to the predetermined values, thereby it becoming possible to form types of posterior dynamic screw (1) that also permit controlled rotation.

The cap (4) Illustrated in Figure-5 and responsible for the prevention of the movement of the screw (3) is formed in a shape extended on both sides in a manner longitudinal with respect to the shape of the surface here, in order to enable it to perfectly fit onto the smooth surfaces formed on the screw (3.1). Moreover, in the upper section of said cap (4), the angle is provided in harmony with the surface geometry of the fixing member (5) formed in cylindrical shape and the both sides are extended such that they will be seated on this cylindrically shaped surface. Another duty of said cap (4) is to prevent the screw (3) from coming out of the body (2), while also ensuring that its movement will remain within the predetermined values.

It is possible to use a rod instead of the fixing member (5) shown in Figure-6, and to use setscrews instead of the tightening member (6), and the function of said fixing member (6) to secure together the posterior dynamic screws (1), which are connected to different spinal columns (7), Said fixing member (5) is positioned by way of engagement into the fixing member slot (2.4) formed on the body (2). The process of tightening said fixing member (5) at its location is achieved by means of the tightening member (6).

The tightening member (6) shown in Figure-7 is connected with the upper part of the body (2), and it is engaged Into the tightening member slot (2.3) formed on the inside of the body (2), by means of the threads formed on the outside thereof. The task of said tightening member (6) is to provide the stabilisation of the fixing member (5) by locking the same.

The body (2), which constitutes the basis for the ability of movement of the posterior dynamic screw (1) shown in Figure-8 and is designed with the shape of a tulip, holds together all the parts, as well as serving as a protective case. After the screw (3) is connected with said body (2), it is positioned such that the head (3.1) part will remain inside the body (2). On the other hand, the cap (4) positioned on said head (3.1) is secured at its location so that it will not dislocate, by means of the fixing member (5) placed on its upper section and positioned by way of connection with the fixing member slot (2.4). Moreover, the spherical head slot (2.2) formed in the bottom section said body (2) enables the cap (4) and the head (3.1) to be positioned in a balanced and appropriate manner.

One of the features of the posterior dynamic screw (1) illustrated in Figure-9 is the buffer (2.1) section formed on the surface of the body (2) close to the vertebra (7). Extending towards the vertebra (7) with a diameter narrower as compared to said body (2), the duty of this buffer (2.1) is to prevent the bone tissue formed, as a result of the operation, between the vertebra (7) and the posterior dynamic screw (1) from reaching the body (2) to cause the ability of the movement to be adversely affected. In other words, the task of the buffer (2.1) is, owing to the special design of the body (2) of the posterior dynamic screw (1) extending in the downward direction, to prevent the bone formation occurring on the surface of the posterior dynamic screw (1) that contacts the vertebra (7) from causing a reduction in the ability of movement, during the period following the implantation. Said posterior dynamic screw (1) is provided with the ability of movement owing to the special designs of the head (3.1) and the cap (4) parts. Both sides of said spherical head (3.1) are smoothened by way of shaving, and the same structure is also formed in the cap (4) part. The assembly is achieved by means of the punching process carried out in the bottom section of said (2) following the establishment of the connection between the cap (4) and the head (3.1), thereby bringing the screw (3) into a state in which it is capable of moving along only a single axis. Since the connection is provided without leaving any gap between the head (3.1) whose side surfaces are smoothened by way of shaving and the cap (4) that is shaved in a way compliant with this head (3.1), the rotation likely to occur on the spinal column is prevented. One of the issues to be taken into account during the assembly of said posterior dynamic screw (1) is to leave such a distance between the head (3.1) and the cap (4) that no opening will be allowed on the envisaged axis.

The protective scope of this application is determined in the section of the claims, and the scope may by no means be limited to the description above provided only for exemplary purposes. It is obvious that a person skilled in the art may provide the innovation put forward by the invention also by modifying the shapes of the parts and may apply similar embodiments to the other fields used for similar purposes. Consequently, such embodiments would obviously lack the criterion of innovative step.

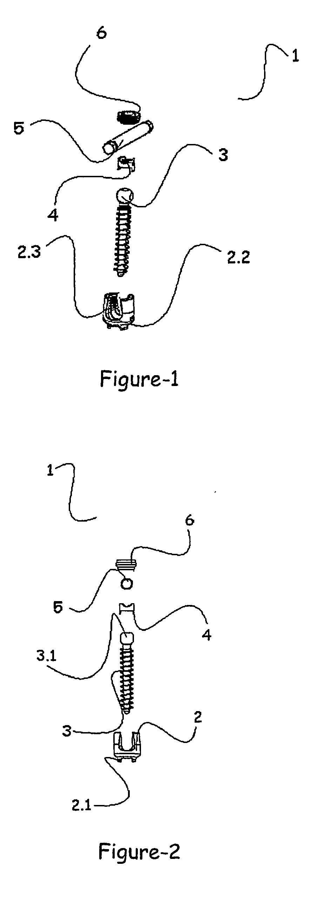

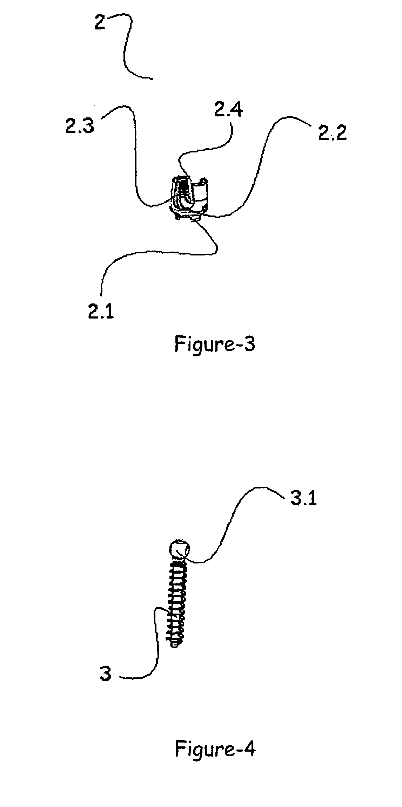

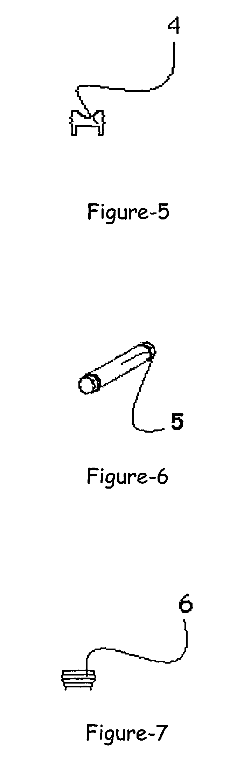

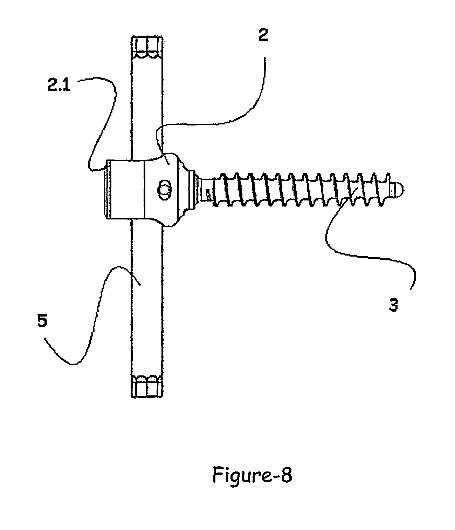

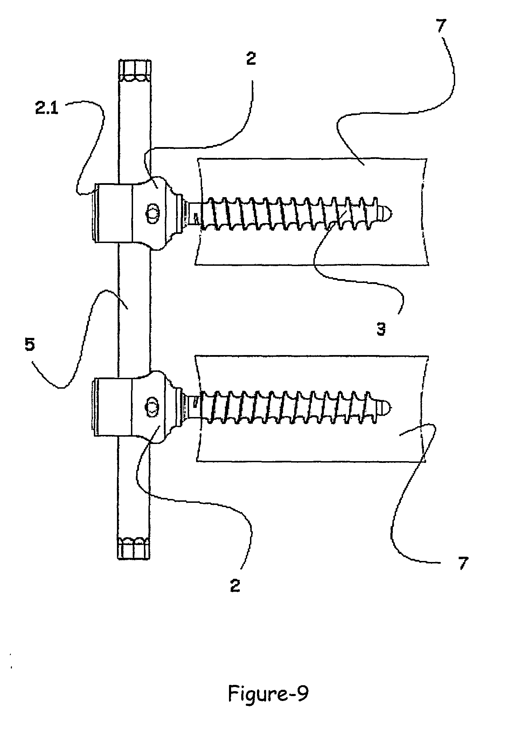

## Claims

1. Posterior dynamic screw (1) to provide the individual with the regained ability of movement by treating the aching spinal column disorders, posterior dynamic screw (1) connected to a vertebra (7) and to be capable of movement in the axial direction comprising:
- a screw (3) with a head (3.1) having a spherical shape and two flat surfaces formed by way of smoothing both sides of the head
- a body (2) for receiving the head (3.1) of the screw (3), wherein the body provides the ability of movement in a single plane while holding together all the parts
- a cap (4), which has its side surfaces smoothened on the inside in a way compare with the surface shape of said head (3.1) and is responsible for preventing the screw (3) from movement
- a fixing member (5)
**characterized in that** the body (2) comprises a protrusion (2.1) In the lower section of said body (2) facing the vertebra to enable the preservation of the ability of movement of said screw (3) with said body (2).

## Patentansprüche

1. Dorsale dynamische Schraube (1), die bei einem Individuum zur Wiedererlangung der Bewegungsfähigkeit für die Behandlung der schmerzenden Wirbelsäulenerkrankungen vorgesehen ist, wobei die dorsale dynamische Schraube (1) mit dem Rückenwirbel (7) verbunden und in der Lage ist, sich in axialer Richtung zu bewegen, und Folgendes aufweist:
- eine Schraube (3) mit einem Kopf (3.1), der eine Kugelgestalt und zwei flache Flächen aufweist, die durch Abflachen der beiden Seiten von dem Kopf geformt sind;
- eine Kopfhalterung (2) zur Aufnahme von dem Kopf (3.1) der Schraube (3), wobei die Kopfhalterung die Fähigkeit zur Bewegung in einer einzelnen Ebene vorsieht, während alle Bauteile zusammengehalten werden;
- eine Abdeckkappe (4), deren Gleitfläche auf der Innenseite derart abgeflacht wurde, dass sie mit der Oberflächenform des Kopfes (3.1) übereinstimmt und die für die Verhinderung der Bewegung der Schraube (3) zuständig ist;
- ein Befestigungselement (5);
**dadurch gekennzeichnet, dass** die Kopfhalterung (2) einen Vorsprung (2.1) in dem unteren Abschnitt der Kopfhalterung (2) aufweist, der dem Rückenwirbel zugewendet ist, um die Erhaltung der Bewegungsfähigkeit der Schraube (3) mit der Kopfhalterung (2) zu ermöglichen.

## Revendications

1. Vis dynamique postérieure (1) permettant à l'individu de regagner une aptitude au mouvement en traitant les troubles douloureux de sa colonne vertébrale, la vis dynamique postérieure (1) étant connectée à une vertèbre (7) et devant être capable de mouvement dans le sens axial, comprenant :
- une vis (3) dotée d'une tête (3.1) ayant une forme sphérique et deux surfaces plates formées en lissant les deux faces de la tête,
- un corps (2) destiné à recevoir la tête (3.1) de la vis (3), le corps permettant l'aptitude au mouvement dans un seul plan tout en tenant toutes les pièces ensemble,
- un capuchon (4) dont les surfaces latérales sont lissées sur l'intérieur de manière compatible avec la forme de la surface de ladite tête (3.1) et qui a pour fonction d'empêcher la vis (3) de bouger,
- un élément de fixation (5),
**caractérisée en ce que** le corps (2) comprend une saillie (2.1) dans sa partie inférieure faisant face à la vertèbre pour permettre la préservation de l'aptitude au mouvement de ladite vis (3) avec ledit corps (2).
